# EUROPEAN PATENT APPLICATION

(11) **EP 2 364 977 A1**
(43) Date of publication of application: **14.09.2011**
(21) Application number: 11151954.2
(22) Date of filing: 25.01.2011
(51) Int. Cl.: C07D 295/135

(54) **Process for the enantiomeric enrichment of 3-methyl-1-(2-piperidinophenyl)-1-butylamine**

(30) Priority: 26.01.2010 EP 10151716
(71) Applicant: Reuter Chemische Apparatebau KG, 79108 Freiburg (DE)
(72) Inventor: Reuter, Karl, 79194, Gundelfingen (DE); Stolz, Florian, 79211, Denzlingen (DE)
(74) Representative: Reitstötter - Kinzebach

(57) **Abstract**

The present invention relates to a process for the enantiomeric enrichment of 3-methyl-1-(2-piperidinophenyl)-1-butylamine (hereinafter PPA), the process comprising the fractional crystallization of PPA in the form of its acid-addition salt with an achiral carboxylic acid of the formula A, in particular a carboxylic acid of the formula A' as described herein from a solution or emulsion containing a mixture of the enantiomers of PPA and the achiral carboxylic acid of the formula A, preferably in the presence of seed crystals of the acid addition salt of the desired enantiomer of PPA with the achiral carboxylic acid of the formula A, whereby the acid-addition salt of PPA with the achiral carboxylic acid of the formula A is obtained, which is enantiomerically enriched with regard to the desired enantiomer of PPA. 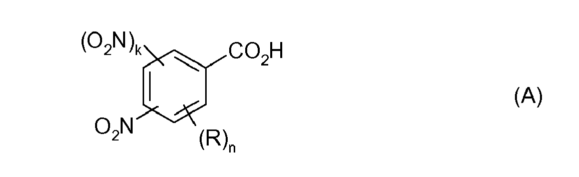 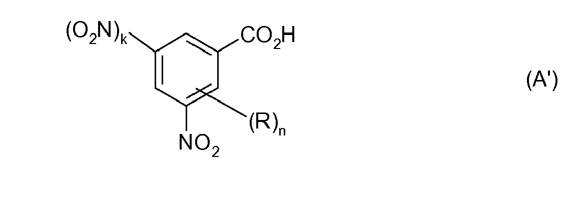 wherein n is 0 or 1, k is 0 or 1 and wherein R is C₁-C₄-alkyl, C₁-C₄-alkoxy and halogen, in particular methyl, methoxy or chlorine.

## Description

The present invention relates to a process for the enantiomeric enrichment of 3-methyl-1-(2-piperidinophenyl)-1-butylamine, in particular of the S-enantiomer (S)-3-methyl-1-(2-piperidinophenyl)-1-butylamine and the acid-addition salts thereof. The invention also relates to specific acid-addition salts of 3-methyl-1-(2-piperidinophenyl)-1-butylamine (hereinafter also PPA) that are useful for obtaining single enantiomers of these compounds.

### BACKGROUND OF THE INVENTION

(S)-3-Methyl-1-(2-piperidinophenyl)-1-butylamine is a valuable precursor in the preparation of Repaglinide which is an oral blood glucose lowering drug of the meglitinide class used in the management of type 2 diabetes mellitus (also known as non-insulin dependent diabetes mellitus or NIDDM). Repaglinide is the INN name of (+)-2-ethoxy-4-[N-[1-(S)-(2-piperidinophenyl)-3-methyl-1-butyl]aminocarbonylmethyl]benzoic acid, which is represented by the following formula

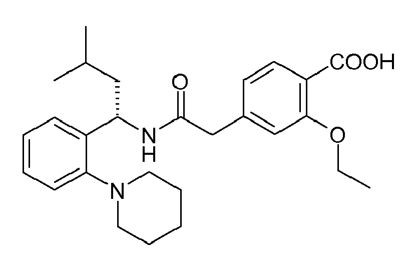

Several approaches for the preparation of Repaglinide have been described in the art. Most of them include the amide forming reaction of 3-methyl-1-(2-piperidinophenyl)-1-butylamine with 4-alkoxycarbonyl-3-ethoxyphenylacetic acid or another 4-carboxyl protected 3-ethoxyphenylacetic acid derivative followed by deprotection. As the amide forming reaction does not affect the configuration of the asymmetric carbon atom in 3-methyl-1-(2-piperidinophenyl)-1-butylamin, much effort has been spent to provide the pure (S) enantiomer of 3-methyl-1-(2-piperidinophenyl)-1-butylamine (hereinafter (S)-PPA).

EP 965591 discloses resolution of racemic 3-methyl-1-(2-piperidinophenyl)-1-butylamine into its enantiomers including the crystallization of the diastereomeric salt of S-3-methyl-1-(2-piperidinophenyl)-1-butylamine with N-acetyl-L-glutamic acid. However, an expensive chiral reagent is required and yields are not satisfactory.

Grell et al, J. Med. Chem., 41 (1998) pp 5219-5246 disclose several approaches to (S)-3-methyl-1-(2-piperidinophenyl)-1-butylamine including the formation of a prochiral precursor of 3-methyl-1-(2-piperidinophenyl)-1-butylamine, enantio-selective reaction of the prochiral precursor with a chiral reagent to obtain a chiral precursor of 3-methyl-1-(2-piperidinophenyl)-1-butylamine and transferring the chiral precursor in a one or two-step reaction sequence into (S)-3-methyl-1-(2-piperidinophenyl)-1-butylamine. These methods, however, are tedious and require the use of expensive chiral reagents.

EP 2019097 discloses resolution of racemic 3-methyl-1-(2-piperidinophenyl)-1-butylamine into its enantiomers including the crystallization of the diastereomeric salt of S-3-methyl-1-(2-piperidinophenyl)-1-butylamine with (4R)-1-benzoyl-4-hydroxy-L-proline. However, an expensive chiral reagent is required and the overall yield is not satisfactory.

WO 2009/04485 discloses resolution of racemic 3-methyl-1-(2-piperidinophenyl)-1-butylamine into its enantiomers including the crystallization of the diastereomeric salt of S-3-methyl-1-(2-piperidinophenyl)-1-butylamine with di-p-toluoyl-D-tartaric acid. However, an expensive chiral reagent is required and the overall yield is not satisfactory.

Therefore, there is still a strong need for providing a process for enantiomeric (optical) enrichment of PPA, in particular of (S)-PPA, that overcomes the disadvantages of the prior art.

### SUMMARY OF THE INVENTION

Surprisingly, it has been found that this object is solved by "preferential crystallization" of acid-addition salts of PPA as defined herein with an achiral carboxylic acid A that is selected from compounds of the formula A, in particular from the formula A': 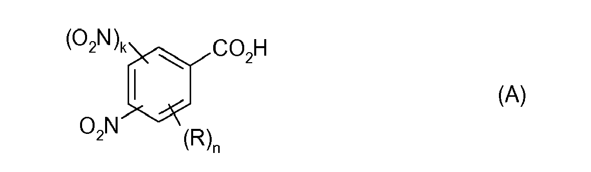 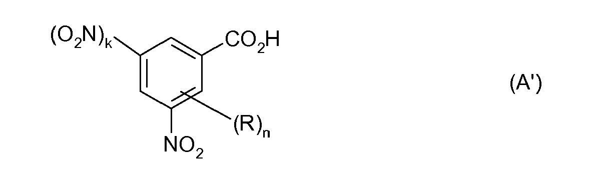 wherein n is 0 or 1, k is 0 or 1 and wherein R is C₁-C₄-alkyl, C₁-C₄-alkoxy and halogen, in particular methyl, methoxy or chlorine.

The preferential crystallization includes the fractional crystallization of PPA in the form of its acid-addition salt with the achiral carboxylic acid of the formulae A or A' (hereinafter also termed achiral carboxylic acid A) from a solution containing a mixture of the enantiomers of the compound of PPA and the achiral carboxylic acid A, preferably in the presence of seed crystals of the acid addition salt of the desired enantiomer of PPA with the achiral carboxylic acid A. In this manner, selective crystallization of the desired enantiomer of PPA as its acid addition salt with the achiral carboxylic acid A is achieved, the enantiomeric ratio of desired enantiomer to non desired enantiomer in isolated material being generally at least 65:35, thus allowing further purification by simple recrystallization of the acid addition salt of PPA with the achiral carboxylic acid A.

Therefore, the present invention relates to a process for the enantiomeric enrichment of PPA, the process comprising the fractional crystallization of PPA in the form of its acid-addition salt with an achiral carboxylic acid A as described herein from a solution or emulsion containing a mixture of the enantiomers of PPA and the achiral carboxylic acid A, preferably in the presence of seed crystals of the acid addition salt of the desired enantiomer of PPA with the achiral carboxylic acid A, whereby the acid-addition salt of PPA with the achiral carboxylic acid A is obtained, which is enantiomerically enriched with regard to the desired enantiomer of PPA.

The term "desired enantiomer" means the one enantiomer, which is to be crystallized first from the solution or slurry.

The invention also relates to the novel acid-addition salts of PPA with said carboxylic acid A, in particular to enantiomerically enriched or pure acid-addition salts of PPA with the carboxylic acid of the formulae A or A' respectively. A particular embodiment of the invention relates to the acid-addition salt of PPA with 4-methyl-3,5-dinitrobenzoic acid, in particular to the acid-addition salt of PPA with 4-methyl-3,5-dinitrobenzoic acid which is enantiomerically enriched. Another particular embodiment of the invention relates to the acid-addition salt of PPA with 3,5-dinitrobenzoic acid, in particular to the acid-addition salt of PPA with 3,5-dinitrobenzoic acid which is enantiomerically enriched.

In this context, enantiomeric enrichment means that the enantiomeric excess (ee) with regard to one enantiomer of PPA in the acid addition salt is at least 30 %, frequently at least 40 %, in particular at least 50 % or at least 60 %. Particular embodiments relate to enantiomerically enriched acid addition salts of PPA with the carboxylic acid of the formulae A or A' respectively, wherein the enantiomeric excess (ee) with regard to one enantiomer of PPA in the acid addition salt is 90 % ee or higher such as > 95 % ee or > 98 % ee.

The use of the acid-addition salt of PPA with the carboxylic acid A provides good crystallization yields and satisfactory enantiomeric enrichment generally exceeding 65:35 (ee = 30 %), frequently at least 70 : 30 (ee = 40 %), in particular at least 75:25 (ee = 50 %) or at least 80 : 20 (ee = 60%), without using expensive chiral auxiliaries. Here and below % ee means the enantiomeric excess. Satisfactory enrichment can be obtained without prior enrichment of PPA to be resolved. Yields as high as ≥ 90 % or ≥ 95 % of theory can by achieved by sufficient number of crystallization cycles as described below. The process of the present invention also allows the use of high concentrations of PPA during crystallization without adversely affecting the enantiomeric enrichment. Moreover, the crystallization of the acid-addition salt of PPA can be carried out in an aqueous solution or emulsion, which renders the process more economical, environment-friendly and less risky, since expensive or dangerous organic solvents can be reduced or avoided.

### DETAILED DESCRIPTION OF THE INVENTION

According to the present invention, 3-methyl-1-(2-piperidinophenyl)-1-butylamine (herein also termed as PPA) is crystallized as its acid-addition salt with the achiral carboxylic acid of the general formula A as defined above or a mixture of achiral acids of the formula A.

Amongst the achiral carboxylic acids of the formula A, preference is given to those, wherein k is 1. Preferably at least one nitro group is located in the meta-position with respect to the carboxyl group. Particular preference is given to the carboxylic acids of the general formula A', in particular to those carboxylic acids of the formula A', wherein k is 1.

If R is present, i.e. n is 1, then R does not have a center of chirality. Preferred meanings of R include C₁-C₂-alkyl, chlorine and C₁-C₂-alkoxy.

The term "halogen" as used herein includes e.g. fluorine, chlorine, bromine or iodine, with particular preference given to chlorine.

The term "C₁-C₄-alkyl" as used herein includes linear or branched alkyl groups having from 1 to 4, in particular 1 or 2 carbon atoms (= C₁-C₂-alkyl), such as methyl, ethyl, n-propyl, isopropyl, n-butyl, isobutyl and 1,1-dimethylethyl (= tert.-butyl).

The term "C₁-C₄-alkoxy" as used herein includes linear or branched alkoxy radicals preferably from 1 to 4, in particular 1 or 2 carbon atoms (= C₁-C₂-alkoxy), such as methoxy, ethoxy, n-propoxy, isopropoxy, n-butoxy, isobutoxy und 1,1-dimethylethyloxy.

Amongst the carboxylic acids of the formulae A or A' particular preference is given to 3,5-dinitrobenzoic acid (3,5-DNBA) and to 4-methyl-3,5-dinitrobenzoic acid (3,5-DNTA).

For purposes of the present invention, the achiral carboxylic acid of the general formulae A or A' is generally used in its pure form, i.e. the achiral acid is a single compound of the formulae A or A' having a purity of at least 90 %, in particular at least 95 % by weight, especially at least 98 % by weight. The "%-purity" relates to the amount of the respective achiral acid A in % by weight, based on the total amount of acid material A plus acidic impurities (i.e. acidic compounds which are not described by formula A) contained therein. However, it is also possible to use a mixture of two or more achiral acids of the formulae A or A', which may be distinct e.g. by the position or number of the nitro groups and/or by the radical R. E.g. a mixture of two different compounds A or A' can be used which are identical with regard to the position and number of the nitro groups but which are distinct with regard to the radical R such as a mixture of 3,5-DNBA and 3,5-DNTA.

According to the invention, a solution or emulsion containing a mixture of the enantiomers of PPA and the achiral carboxylic acid A is subjected to a fractional crystallization by analogy to a manner known in the art. Principally, the crystallization of one enantiomer of the acid-addition salt of PPA with the carboxylic acid A will be initiated by seed crystals of the same enantiomer of the acid-addition salt of PPA with the carboxylic acid A, thereby effecting and promoting the crystallization of this enantiomer. The seed crystals may be added to the solution or emulsion containing the mixture of the enantiomers of PPA and the achiral carboxylic acid A. However, it is also possible to generate the seed crystals in-situ, if the solution or emulsion containing the mixture of the enantiomers of PPA and the achiral carboxylic acid A is already enriched (or depleted) with regard to one enantiomer of PPA.

It is also possible to induce fractional crystallization by simultaneous addition to the solution or emulsion containing the mixture of the enantiomers of PPA and the achiral carboxylic acid A of a mixture of two types of seed crystals, where the one type of seed crystals is enriched with the acid addition salt of the one enantiomer of PPA with the achiral acid A while the other type of seed crystals being enriched with the acid addition salt of the other enantiomer of PPA with the achiral acid A, provided that the seed crystals of the one enantiomer lead to a morphology or particle size of the crystallized material of the one enantiomer that is distinct from the morphology or particle size of the crystallized material of the other enantiomer induced by the seed crystals of the other enantiomer. E. g. it is possible to use a mixture of seed crystals, where the particle size of seed crystals of the one enantiomer is significantly larger, e.g. by at least one order of magnitude, than the particle size of the seed crystals of the other enantiomer.

The fractional crystallization of the acid-addition salt of 3-methyl-1-(2-piperidinophenyl)-1-butylamine is preferably carried out in the presence of seed crystals of the desired enantiomer of the acid-addition salt of 3-methyl-1-(2-piperidinophenyl)-1-butylamine with the carboxylic acid A. Thereby the crystallization of the desired enantiomer is initiated and promoted as described herein.

The desired enantiomer is generally the S-enantiomer of 3-methyl-1-(2-piperidino-phenyl)-1-butylamine, hereinafter also termed as S-PPA. The invention, however, can also be used to enrich the R-enantiomer of 3-methyl-1-(2-piperidinophenyl)-1-butylamine (*R*-PPA), which is then the desired enantiomer. If the S-enantiomer is the desired enantiomer, however, the R-enantiomer is the non-desired enantiomer.

Usually, these crystals are provided to the solution containing dissolved the acid addition salt of PPA with the achiral acid A or a mixture of the enantiomers of PPA and the achiral carboxylic acid A by addition of seed crystals of the acid-addition salt of the desired enantiomer to the solution. The seed crystals may be generated in situ, if the desired enantiomer in the mixture to be resolved is present in excess, i.e. if a non racemic mixture of PPA or its acid addition salt is subjected to crystallization.

Preferably, the mixture of enantiomers dissolved in the solution is a racemic mixture or almost racemic mixture of the respective enantiomers of PPA, i.e. that the ratio of the PPA enantiomers is between 40:60 and 60:40, in particular between 45:55 and 55:45, corresponding to an enantiomeric excess of < 20 % ee, in particular < 10 % ee. However, it is also possible to use a non-racemic mixture of the enantiomers of PPA, or of its acid-addition salt, in which the desired enantiomer has already been enriched (or the non-desired enantiomer has been depleted) to a level of more than 20 % ee prior to crystallization. To this non-racemic mixture seed crystals of the desired enantiomer or its acid-addition salt can be added or the seed crystals may be generated in situ, if the desired enantiomer in the mixture to be resolved is present in excess. It is also possible to effect crystallization of the deficit enantiomer (as its acid addition salt with the achiral acid A) from said non racemic mixture or from an almost racemic mixture of the enantiomer by addition of seed crystals of said deficit enantiomer.

In a preferred embodiment of the invention, crystallization is effected from a solution or emulsion of the racemic or almost racemic mixture of PPA in the form of its acid-addition salt with the achiral acid A by addition of seed crystals of the acid addition salt of the desired enantiomer with said achiral acid A.

In another preferred embodiment of the invention, crystallization is effected from a solution, suspension or emulsion of the non-racemic mixture of PPA in the form of its acid-addition salt with the achiral acid A, where the non-racemic mixture contains an excess of the desired enantiomer of PPA. By this, the enantiomeric enrichment of the desired enantiomer in the obtained crystalline material can be easily increased to values of 90 % ee or higher such as > 95 % ee or > 98 % ee.

If a non-racemic mixture of the enantiomers of PPA is used for crystallization, the excess of the desired enantiomer generally does not exceed 50 % ee, i. e. the molar ratio of desired enantiomer to non-desired enantiomer does not exceed 75 : 25. Such non-racemic mixtures may be obtained during crystallization of the acid-addition salt of the desired enantiomer from a solution of the racemic mixture of PPA according to the process of the present invention. During this process, the desired enantiomer is depleted and a non-racemic mixture of the enantiomers of PPA remains in the mother liquor. The mother liquor or the non-racemic mixture of enantiomers isolated therefrom may be used in a subsequent crystallization of the acid-addition salt of the other enantiomer. Preferably, seed crystals are used for the crystallization of the racemic or almost mixture of PPA (ee < 20 %), while for crystallization of non-racemic mixtures of PPA having an ee > 20 %, the seed crystals may be generated in situ. It is, however, also possible to add seed crystals of the desired enantiomer to a solution or emulsion of the non-racemic mixture containing an excess of the desired enantiomer. It is also possible to add seed crystals of the non-desired enantiomer to a solution of the non-racemic mixture containing an excess of the desired enantiomer, thereby effecting crystallization of the non-desired enantiomer.

As pointed out above, it is also possible to subject a non-racemic mixture of the enantiomers of PPA in the form of their acid addition salt with the achiral carboxylic acid to the process of the invention, where the desired enantiomer is present in larger excess, e.g. where the enantiomeric excess is > 50 % ee, e.g. from > 50 to 99 % ee. This allows further enantiomeric enrichment of the desired enantiomer.

As pointed out above, it is also possible to subject a racemic or almost racemic mixture of the enantiomers of PPA in the form of their acid addition salt with the achiral carboxylic acid A to a fractional crystallization comprising the addition of a mixture of two types of seed crystals of both enantiomers of the acid addition salt of PPA with the achiral acid A to the solution or emulsion containing the racemic or almost racemic mixture of the enantiomers of PPA and the achiral acid A. For this purpose, the properties of the respective seed crystals of the acid addition salts of the one enantiomer and of seed crystals of the acid addition salts of the other enantiomer are chosen in a manner that they will lead to a morphology or particle size of the crystallized material of the one enantiomer that is distinct from the morphology or particle size of the crystallized material of the other enantiomer. In particular the seed crystals of the acid addition salts of the respective enantiomers will differ with regard to their particle size, preferably by a factor of at least 10, in particular by a factor of at least 20, e.g. by a factor from 10 to 1000, in particular by a factor of from 20 to 500. Thereby, the crystallization will lead to a conglomerate of crystals of different particle sizes, where the crystals containing an excess of the enantiomer enriched in the large seed crystals will have a particle size which is significantly greater than the particle size of the crystals containing an excess of the enantiomer enriched in the smaller seed crystals. These crystals can be fractionated with regard to their particle size and thus with regard to the enantiomers contained therein by standard techniques for fractionating particulate materials, e.g. by sieving or by sedimentation.

According to the invention, the crystallization of the acid-addition salt of PPA is performed in the presence of the carboxylic acid A. Needless to say, the amount of carboxylic acid A is chosen to ensure crystallization of the acid-addition salt of PPA. Usually, the amount of achiral carboxylic acid A present is close to the required stoichiometry of the acid-addition salt of compound I and generally does not deviate from the required stoichiometry by more than 20 mol-%. In particular, the molar ratio of carboxylic acid A to PPA in the solution before crystallization is from 0.9 : 1 to 1.1 : 1, in particular from 0.95 : 1 to 1.05 : 1. The necessary amount of the carboxylic acid A can be added to a solution of PPA or vice versa. Thereby, a solution of the acid-addition salt is formed. Alternatively, a solution containing PPA and the carboxylic acid A can be provided by dissolving the acid-addition salt of PPA with the achiral carboxylic acid A. The total concentration of PPA and the achiral carboxylic acid A in the solution or emulsion before crystallization is usually from 25 to 700 g/L, preferably from 50 to 650 g/L, in particular from 75 to 500 g/L, or 100 to 400 g/L.

The crystallization of the acid-addition salt of PPA can be performed by analogy to well-known crystallization techniques. Their basic premise is the formation of a solution containing the mixture of the enantiomers of the compound of PPA and the achiral carboxylic acid A, supersaturation by conventional techniques, and subsequent induction of the crystallization of the desired enantiomer. Induction is achieved by the presence of seed crystals of the acid addition salt of desired enantiomer of PPA with the achiral acid A. Induction can be achieved by adding seed crystals of the acid addition salt of desired enantiomer or by "self seeding" e.g. by further lowering the solubility of the acid-addition salt and/or by scratching or ultrasound if the mixture to be crystallized is non-racemic and the desired enantiomer is present in excess. In this manner, seed crystals of the acid addition salt of the desired enantiomer are formed in situ. Lowering the solubility of the acid-addition salt can be achieved by lowering the temperature, adding a non-solvent to the solution and/or by adding further achiral carboxylic acid A or PPA.

Preferably, crystallization is induced by addition of seed crystals of the acid-addition salt of the desired enantiomer. The amount of seed crystals added to the solution of the mixture of enantiomers is preferably from 0.1 to 100 mmol, preferably from 0.2 to 10 mmol, in particular from 0.3 to 5 mmol, per mol of the compound of PPA contained in the solution.

In one embodiment of the invention the crystallization is performed in the presence of at least one surface active compound, which, below, is also termed as surfactant. Suitable surfactants include non-ionic, anionic, cationic and zwitterionic surfactants and mixtures thereof. In a preferred embodiment, the surfactant will be present in an amount ranging from 0.1 to 30 % by weight, based on the total weight of the solution before crystallization, in particular from 0.5 to 20 % by weight.

Anionic surfactants include in particular the sodium, potassium calcium or ammonium salts of
- C₆-C₂₂-alkylsulfonates such as lauryl sulfonate, isotridecylsulfonate;
- C₆-C₂₂-alkylsulfates such as lauryl sulfate, isotridecylsulfate, cetylsulfate, stearylsulfate;
- aryl- and C₁-C₁₆-alkylarylsulfonates such as naphthalene sulfonate, dibutylnaphthalene sulfonate, dodecyldiphenylether sulfonate, cumylsulfonate, nonylbenzenesulfonate, dodecylbenzene sulfonate;
- sulfates and sulfonates of fatty acids and fatty acid esters;
- sulfates of ethoxylated C₆-C₂₂ alkanoles such as sulfates of ethoxylated lauryl alcohol;
- sulfates of ethoxylated C₁-C₁₆-alkylphenols;
- mono- and diesters of phosphorous acid, including mixtures thereof with triesters and salts thereof, in particular the esters with C₈-C₂₂-alkanols, ethoxylated C₈-C₂₂-alkanols, C₄-C₂₂-alkylphenols, ethoxylated C₄-C₂₂-alkylphenols, di- or tristyrylphenols, ethoxylated di- or tristyrylphenols;
- di C₄-C₁₆ alkylesters of sulfosuccinic acid such as dioctylsulfosuccinate;
- acylsarcosinates;
- fatty acids such as stearates;
- acylglutamates; and
- condensates of naphthalinesulfonic acid or phenolsulfonic acid with
   formaldehyde.
Non-ionic surfactants include in particular
- polyethyleneglycolalkylethers, polyethyleneglycol/polypropyleneglycolalkylethers, in particular polyethoxylates and poly-ethoxylates-co-propoxylates of linear or branched C₈-C₂₀-alkanoles, more preferably polyethoxylated fatty alcohols and polyethoxylated oxoalcohols, such as polyethoxylated lauryl alcohol, polyethoxylated isotridecanol, polyethoxylated cetyl alcohol, polyethoxylated stearyl alcohol, and esters thereof, such as acetates;
- polyethylenglycol arylethers, in particular polyethoxylates of C₁-C₁₆-alkylphenoles, such as polyethoxylates of nonylphenol, decylphenol, isodecylphenol, dodecylphenol or isotridecylphenol, polyethoxylates of mono-, di-und tristyrylphenoles; and the esters thereof, e.g. the acetates;
- C₆-C₂₂-alkylglucosides and C₆-C₂₂-alkyl polyglucosides;
- polyethoxylates of C₆-C₂₂-alkylglucosides and polyethoxylates of C₆-C₂₂-alkyl polyglucosides;
- polyethoxylates of fatty amines;
- polyethoxylates of fatty acids and polyethoxylates of hydroxyl fatty acids;
- partial esters of polyols with C₆-C₂₂-alkanoic acids, in particular mono- and diesters of glycerine and mono-, di- and triesters of sorbitan, such as glycerine monostearate, sorbitanmonooleat, sorbitantristearat;
- polyethoxylates of partial esters of polyols with C₆-C₂₂-alkanoic acids, in particular polyethoxylates of mono- and diesters of glycerine and polyethoxylates of mono-, di- and triesters of sorbitan, such as polyethoxylates of glycerine monostearate, polyethoxylates of sorbitanmonooleat, polyethoxylates of sorbitanmonostearat and polyethoxylates of sorbitantristearat;
- polyethoxylates of vegetable oils or animal fats such as corn oil ethoxylate, castor oil ethoxylate, tallow oil ethoxylate;
- triesters of phosphorous acid, in particular the triesters with C₈-C₂₂-alkanols, ethoxylated C₈-C₂₂-alkanols, C₄-C₂₂-alkylphenols, ethoxylated C₄-C₂₂-alkylphenols, di- or tristyrylphenols, ethoxylated di- or tristyrylphenols;
- polyoxyethylene-polyoxypropylene-blockcopolymers; and
- polyethoxylates of fatty amines, fatty amides or of fatty acid diethanolamides. Cationic surfactants include in particular
- protonated mono-, di- and trialkylammonium compounds and quaternary tetralkylammonium compounds, in particular C₆-C₂₂-alkyltrimethylammonium salts and di-C₆-C₂₂-alkyldimethylammonium salts, e.g. the halides, sulfates and alkylsulfates, including protonated or quarternized polyethoxylates of mono-, di-and trialkylamines;
- alkyl pyridinium salts, in particular C₆-C₂₂-alkylpyridinium salts e.g. the halides, sulfates and C₁-C₄-alkylsulfates; and
- alkyl and dialkyl imidazolinium salts, in particular N,N'- C₆-C₂₂-dialkylimidazolinium salts, e.g. the halides, sulfates or methoxysulfates.
Zwitterionic (amphoteric) surfactants include in particular
- aminoxides of tertiary alkylamines; and
- betaines, in particular 2-(trialkylamonium)acetic acid or 2-(N-(alkylcarbonylaminoalkyl)-N,N-dialkylamino)acetic acid or coco-beta-aminobutyric acid.

The terms polyethyleneglycol and polyethoxylates refer to polyether radicals derived from ethyleneoxide. Likewise, the term polyoxyethylene-co-polyoxypropylene refers to a polyether radical derived from a mixture of ethyleneoxide and propylenoxide. The number of repeating units in the polyether radicals will generally vary from 2 to 100, in particular from 4 to 50.

Preferably, the surfactant, if present, comprises at least 50 % by weight, based on the total amount of surfactant present, of at least one non-ionic surfactant. In particular, the surfactant comprises at least 80 %, based on the total amount of surfactant, of at least one non-ionic surfactant.

Among the non-ionic surfactants preferred are those which have an HLB-value of at least 5, in particular from 5 to 20.

Preferred non-ionic surfactants include
- polyethyleneglycolalkylethers, polyethyleneglycol/polypropyleneglycolalkylethers, in particular polyethoxylates and poly-ethoxylates-co-propoxylates of linear or branched C₈-C₂₀-alkanoles, more preferably polyethoxylated fatty alcohols and polyethoxylated oxoalcohols, such as polyethoxylated lauryl alcohol, polyethoxylated isotridecanol, polyethoxylated cetyl alcohol, polyethoxylated stearyl alcohol;
- polyethylenglycol arylethers, in particular polyethoxylates of C₁-C₁₆-alkylphenoles, such as polyethoxylates of nonylphenol, decylphenol, isodecylphenol, dodecylphenol or isotridecylphenol, polyethoxylates of mono-, di-und tristyrylphenoles;
- polyoxyethylenesorbitanesters of C₈-C₂₂-aliphatic acids, such as polyethoxylates of sorbitanmonooleat, polyethoxylates of sorbitanmonostearat and polyethoxylates of sorbitantristearat;
- polyethoxylates of vegetable oils or animal fats, such as corn oil ethoxylate, castor oil ethoxylate, tallow oil ethoxylate;
- polyethoxylates of mono- or diglycerides of fatty acids, such as polyethoxylates of glycerine monooleate or glycerine monostearate;
- polyoxyethylene-polyoxypropylene-blockcopolymers; and
- polyethoxylates of fatty acids and polyethoxylates of hydroxyl fatty acids such as polyethoxylates of hydroxystearic acid.

Particular preference is given to polyethyleneglycolsorbitan fatty acid esters, polyethyleneglycol C₈-C₂₀-alkylethers, polyethoxylates of C₄-C₁₀-alkylphenoles, ethoxylated castor oil and polyethyleneglycolhydroxystearat.

According to the invention, the crystallization of the acid addition salt is performed from a solution of the mixture of enantiomers of PPA and the carboxylic acid A, i.e. the mixture of enantiomers of PPA and the carboxylic acid A, either as separate compounds or in the form of the acid addition salt of PPA with the achiral carboxylic acid, are dissolved in a suitable solvent or solvent mixture prior to crystallization. The solution can be a homogenous solution, i.e. the mixture of enantiomers of PPA, the carboxylic acid A and at least one solvent form a single phase prior to crystallization, or a heterogeneous solution or emulsion, wherein the mixture of enantiomers, the carboxylic acid A are dissolved in at least one solvent, preferably in a mixture of at least two solvents of different polarity, thereby forming a multi-phase liquid. Thus, the crystallization can be performed either from a homogeneous solution containing the mixture of the enantiomers of PPA and the achiral carboxylic acid A in a suitable solvent, or from an emulsion in which the mixture of the enantiomers of PPA and the achiral carboxylic acid A are dissolved. Both, the homogenous solution and the emulsion may further comprise solid material, in particular undissolved carboxylic acid A and/or PPA or inert solid material, which is insoluble or sparingly soluble in the solvent of choice.

For crystallization from a homogeneous solution, the mixture of the enantiomers of PPA, either in the form of its free base or in the form of its acid-addition salt, is dissolved in a suitable solvent. If the free base is used, the necessary amount of the achiral carboxylic acid A is added to the solution.

Suitable solvents include in particular organic solvents that have a miscibility with water of at least 10 % by weight at room temperature, and mixtures thereof with water. Likewise mixtures of organic solvents having a miscibility with water of at least 10 % by weight at room temperature with organic solvents having a miscibility with water of less than 10 % by weight at room temperature, as well as mixtures thereof with water, can be used as a solvent for crystallization.

Suitable organic solvents that have a miscibility with water of at least 10 % by weight at room temperature include:
1. C₁-C₅-alkanols such as methanol, ethanol, n-propanol, isopropanol, n-butanol, isobutanol, tert.-butanol, n-pentanol etc.;
2. amides and N,N-dimethylamides of C₁-C₃-carboxylic acids such as formamide, dimethylformamide (DMF), acetamide and N,N-dimethylacetamide;
3. 5 or 6-membered lactames with a total of 7 carbon atoms such as pyrrolidone, N-methylpyrrolidone, N-ethylpyrrolidone, N-isopropylpyrrolidone, N-hydroxyethylpyrrolidone;
4. dimethylsulfoxid and sulfolane;
5. acetonitrile;
6. 5- or 6-membered lactones such as γ-butyrolactone;
7. polyols and polyetherols such as glycol, glycerin, dimethoxyethan, ethylendiglycol, ethylenglycolmonomethylether, etc.

Suitable organic solvents that have a miscibility with water of less than 10 % by weight at room temperature include:
8. aromatic solvents, such as benzene or its derivatives like toluene, benzonitrile, nitrobenzene, chlorobenzene or xylene and heteroaromatic liquids such as pyridine or furane;
9. halogenated alkanes like dichloromethane, dichloroethane, trichloroethane;
10. alkyl ethers, with ≥ 4 carbon atoms, such as diethylether or tert-butylmethylether;
11. esters such as n-, i- or branched with ≥ 5 carbon atoms, including diesters, triesters, such as oils and fats, and polyesters;
12. alkanols, aromatic and cyclic alcohols with ≥ 6 carbon atoms, in particular 6 to 10 carbon atoms, e.g. 2-hexanol, cyclohexanol, benzylalcohol or octanol;
13. alkanes such as n-, i- or branched, including cycloalkanes (e.g. cyclohexane, methylcyclohexane).

In a preferred embodiment of the invention, the crystallization of the acid-addition salt of PPA is performed from a homogeneous solution containing the mixture of enantiomers and the carboxylic acid A in a C₁-C₅-alkanol, like methanol, ethanol, n-propanol, isopropanol, n-butanol, isobutanol, tert.-butanol, n-pentanol, or a mixture thereof with another solvent having a miscibility with water of at least 10 % at room temperature or a mixture thereof with water containing at least 50 % v/v, in particular at least 80 % v/v of organic solvents.

In another preferred embodiment of the invention, the crystallization of the acid-addition salt PPA is performed from a homogeneous solution containing the mixture of enantiomers and the carboxylic acid A in a mixture comprising at least one solvent having a miscibility with water of at least than 10 % by weight at room temperature, in particular at least one solvent of the groups 1, 3 and/or 6 with at least one solvent having a miscibility with water of less than 10 % by weight at room temperature, in particular a solvent of the group 12.

For crystallization from a homogeneous solution, the solution of the mixture of the enantiomers of PPA and the necessary amount of achiral carboxylic acid A is supersaturated, and then crystallization is induced as described above.

Supersaturation can be achieved by standard methods, for example by dissolving PPA and the achiral carboxylic acid A (or the acid addition salt of PPA with the achiral carboxylic acid A) in the respective solvent or solvent mixture with heating in an amount so that the obtained solution is saturated or nearly saturated. Then, the obtained solution is cooled down below the saturation temperature, whereby a supersaturated solution is obtained. Likewise, supersaturation can be achieved by evaporating solvent from a non-saturated solution until the concentration of the acid-addition salt of PPA is higher than the concentration of a saturated solution. It is likewise possible to obtain a supersaturated solution by adding more achiral carboxylic acid A and/or PPA or the acid addition salt of PPA with the achiral carboxylic acid in order to reduce the solubility of the acid-addition salt of the enantiomers of PPA in the solution. Of course, any of these measures can be combined.

In the thus obtained supersaturated solution, crystallization is induced as described above. In particular, crystallization is induced by adding seed crystals of the acid-addition salt of the desired enantiomer. The seed crystals can be added as such or in the form of a suspension in the solvent or solvent mixture, respectively. The particle size of the seed crystals may range from 0.1 to 200 µm, preferably from 0.5 to 20 µm, in particular from 0.5 to 10 µm. It can be advantageous to further lower the temperature of the solution after the seed crystals have been added. During crystallization, the temperature can be reduced and/or further solvent can be evaporated in order to assist the crystallization. In a preferred embodiment, the temperature during crystallization is lowered by at least 5 K, preferably at least 10 K, e.g. by 10 to 20 K. Usually, the crystallization process is performed at a temperature ranging from -20 to +80° C, in particular from 0 to 50° C. The crystallization process can be further optimized by a proper agitation during the crystallization, e. g. by stirring, shaking or pumping.

The total concentration of PPA and achiral carboxylic acid A prior to crystallization from the homogeneous solution may vary, depending on the solvent and the carboxylic acid A. Usually, the concentration ranges from 25 to 700 g/L, preferably from 50 to 650 g/L, in particular from 75 to 500 g/L, more preferably from 100 to 400 g/L.

In another preferred embodiment, the crystallization is effected by so-called "emulsion crystallization". Emulsion crystallization is a well-established technique and known e. g. from EP 548028, WO 97/32644, WO 99/12623, WO 00/54865 and WO 00/53283.

In emulsion crystallization, the crystallization of the desired enantiomer of the acid-addition salt is effected from a supersaturated emulsion containing the mixture of the enantiomers of PPA in the form of their acid-addition salts with the carboxylic acid A. The emulsion comprises a continuos phase formed by at least one first solvent and a discontinuous phase (droplets) formed by at least one second solvent. The acid addition salt of the compound of formula I may be present in both phases. The discontinuous phase may be formed by a non-polar (lipophilic) solvent or an amphiphilic solvent or a mixture thereof, while the continuos phase is formed by a highly polar organic solvent or water or a mixture thereof. However, emulsions where the discontinous phase is formed by the polar solvent and the continous phase is formed by the non-polar and/or amphiphilic solvent, are likewise suitable for the purpose of the invention.

The emulsion can be a macroemulsion, i.e. the average droplet size is > 500 nm, in particular > 1 µm, or a microemulsion, i. e. the average droplet size is < 500 nm, in particular < 300 nm. Crystallization from microemulsions is preferred. The droplet size can be determined by light scattering methods.

In the emulsions, the relative phase volume of the disperse phase may vary and preferably lies in a range from 5 to 65 % v/v, in particular from 10 to 50 % v/v, based on the total volume of the emulsion.

The emulsion will usually contain one or more surfactant as described above, which assists in forming and stabilizing the emulsion droplets. The surfactants can be non-ionic, anionic, cationic or zwitterionic. Preferably, the surfactant, if present, comprises at least 50 % by weight, based on the total amount of surfactant present, of at least one non-ionic surfactant. In particular, the surfactant comprises at least 80 %, based on the total amount of surfactant, of at least one non-ionic surfactant. The surfactant will normally be present in an amount ranging from 0.1 to 30 % by weight, based on the total weight of the emulsion, in particular from 0.5 to 20 % by weight.

For obtaining an emulsion containing the enantiomers of the acid-addition salt, said mixture of enantiomers can be dissolved in a suitable mixture of solvents, in particular a mixture of water and organic solvent. The mixture usually contains a surface active agent suitable for stabilizing the droplets of the emulsion. It is likewise possible to dissolve the mixture of enantiomers of the acid-addition salt or the free base of I and the achiral carboxylic acid A in a suitable solvent and then emulsify the obtained solution together with a surfactant in water or another solvent, which is suitable for forming an emulsion.

The non polar solvents are organic solvents having a water solubility of ≤ 5 % v/v at room temperature. Amphiphilic solvents are organic solvents that are soluble in both non-polar, lipophilic and polar, hydrophilic phases. They have a water solubility of > 5 % v/v at room temperature and a solubility of > 5 % v/v at room temperature in methyloleate. Highly polar solvents include water and organic solvents which have a solubility of ≤ 5 % v/v at room temperature in methyloleate.

Suitable solvents for emulsion crystallization include
I. non-polar, lipophilic solvents, such as
   1. aromatic solvents, such as benzene or its derivatives like toluene, benzonitrile, nitrobenzene, chlorobenzene or xylene and heteroaromatic liquids such as pyridine or furane;
   2. halogenated alkanes like dichloromethane, dichloroethane, trichloroethane;
   3. alkyl ethers, with ≥ 4 carbon atoms, such as diethylether or tert-butylmethylether;
   4. esters such as n-, i- or branched with ≥ 5 carbon atoms, including diesters, triesters, such as oils and fats, and polyesters;
   5. alkanoles, aromatic and cyclic alcohols with ≥ 6 carbon atoms, e.g. 2-hexanol, cyclohexanol, benzylalcohol or octanol;
   6. alkanes such as n-, i- or branched, including cycloalkanes (e.g. cyclohexane, methylcyclohexane).
II. amphiphilic solvents, such as
   1. ethers such as tetrahydrofurane, dimethoxyethane, dioxane, trioxane, diethyleneglycoledimethylether, triethyleneglycoledimethylether, dipropyleneglycoldimethylether, low molecular weight polyethyleneglycoles and low molecular weight polypropyleneglycoles (M_{w} ≤ 400);
   2. alkanols and cycloalkanols with 1 to 5 carbon atoms such as methanol, ethanol, isopropanol, isobutanol, cyclobutanol and cyclopentanol;
   3. aminoalcohols such as ethanolamine, diethanolamine and triethanolamine;
   4. ketones with 3 to 6 carbon atoms such as acetone, 2-butanone, cyclopentanone and cyclohexanone;
   5. esters of C₁-C₃-alkanoic acids with C₁-C₄-alkanols having a total ≤ 4 carbon atoms such as methyl and ethyl acetate, methyl propionate, methyl-, ethyl-and propyl formiate, the mono- and diacetates of ethylenglycol, propylenglycol, diethylenglycol or triethylenglycol;
   6. lactones such as γ-butyrolactone;
   7. amides and N,N-dimethylamides of C₁-C₃-carboxylic acids such as formamide, dimethylformamide (DMF), acetamide and N,N-dimethylacetamide;
   8. 5 or 6-membered lactames such as pyrrolidone, N-methylpyrrolidone, N-ethylpyrrolidone, N-isopropylpyrrolidone, N-hydroxyethylpyrrolidone, n-propylpyrrolidone, n-octylpyrrolidone.
III. highly polar solvents, such as water or dimethylsulfoxid (DMSO).

In a preferred embodiment of the emulsion crystallization, the crystallization is performed from an aqueous emulsion, i.e. the emulsion comprises water and at least one organic solvent. The total amount of organic solvents may vary and is preferably from 40 to 95 % w/w, in particular from 60 to 95 % w/w, based on the total amount of organic solvent and water. Preferred organic solvents for emulsion crystallization from an aqueous emulsion are selected from the group of amphiphilic solvents II, in particular from the groups II.2, II.4, II.6, II.7 and II.8.

The emulsion may further contain additives such as anti-foaming agents, solubility regulating additives, anti-freeze agents and the like. Agents for adjusting the solubility and/or the freezing point of the aqueous phase include e.g. a water-miscible organic liquid such as a C₁-C₃-alkanol, dimethyl sulfoxide, acetonitrile, etc.

For crystallization from the emulsion, the emulsion containing the mixture of the enantiomers of PPA and the necessary amount of achiral carboxylic acid A is supersaturated, and then crystallization is induced as described above.

Supersaturation can be achieved in a manner similar to that of the methods described above, preferably by dissolving the mixture of enantiomers of PPA and the achiral carboxylic acid A (or the acid addition salt of PPA with carboxylic acid A) in the respective solvent or solvent mixture with heating in an amount so that the obtained emulsion is saturated or nearly saturated and cooling the obtained emulsion below the saturation temperature, whereby a supersaturated emulsion is obtained. It is likewise possible to obtain a supersaturated emulsion by adding further achiral carboxylic acid A and/or PPA in order to reduce the solubility of the acid-addition salt of the enantiomers of PPA in the emulsion or by dissolving additional acid addition salt of PPA with the achiral acid A by the use of ultrasound or by subsequent addition of the achiral carboxylic acid A and PPA. Of course, any of these measures can be combined.

In the thus obtained supersaturated emulsion crystallization is induced as described above. In particular, crystallization is induced by adding seed crystals of the acid-addition salt of the desired enantiomer to the emulsion. The seed crystals can be added as such or in the form of a suspension in a solvent or in emulsion. The particle size of the seed crystals may range from 0.1 to 200 µm, preferably from 0.5 to 20 µm, in particular from 0.5 to 10 µm. It can be advantageous to further lower the temperature of the emulsion after the seed crystals have been added. During crystallization, the temperature can be reduced and/or further solvent can be evaporated in order to assist the crystallization. In a preferred embodiment, the temperature during emulsion crystallization is kept constant or nearly constant (± 2K). Usually, the crystallization process is performed at a temperature ranging from -20 to +80° C, in particular from 0 to 50° C. The crystallization process can be further optimized by proper agitation during the crystallization, e. g. by stirring, shaking or pumping.

The total concentration of PPA and the achiral carboxylic acid A in the emulsion before crystallization starts may range from 25 to 700 g/L, preferably from 50 to 650 g/L, in particular from 75 to 500 g/L, more preferably from 100 to 400 g/L.

Irrespectively of the crystallization technique used, the crystallization of the desired enantiomer of the acid-addition salt is usually performed such that at least 5 g/L of the desired enantiomer of the acid-addition salt of PPA have been crystallized. Preferably, depending on the carboxylic acid A, the concentration and the level of supersaturation used, the crystallization is performed in such a way, that 5 to 200 g/L, in particular 10 to 100 g/L of the desired enantiomer of the acid-addition salt is obtained.

The crystalline material is separated from the mother liquor by usual solid/liquid separation techniques, including filtration and centrifugation. After the removal of the mother liquor, the obtained solid material is usually washed with water and/or a suitable organic solvent to remove residual mother liquor.

The thus obtained crystalline product usually has an enantiomeric ratio (er) of at least 65 : 35, in particular at least 70 : 30 (weight ratio of the desired enantiomer to non-desired enantiomer as free base or as their acid addition salt with achiral acid A, respectively).

In order to further enrich the desired enantiomer, the obtained crystalline material can be subjected to a further conventional re-crystallization. The re-crystallization of the acid-addition salt can be performed from a homogeneous solution of the acid-addition salt, from a heterogenous solution (i.e. from a suspension) or from an emulsion as described above. In particular, the acid-addition salt obtained from the preferential crystallization is re-crystallized from a solvent, selected from water, organic solvents having a water miscibility of at least 10 % v/v at room temperature and mixtures thereof with water. In a particular embodiment, the solvent used for re-crystallization is selected from C₁-C₄-alkanols such as methanol, ethanol, n-propanol, isopropanol, n-butanol, isobutanol or tert.-butanol, or mixtures thereof with water. In another particular embodiment, the solvent is selected from organic solvents which are less polar than C₁-C₄-alkanols, in particular from ketones having 3 to 8 carbon atoms such as methylethylketone, dialkylethers with ≥ 4 carbon atoms, e.g. with 4 to 8 carbon atoms, such as diethylether, diisopropylether or tert-butylmethylether and aliphatic esters such as n-, i- or branched with ≥ 3 carbon atoms in particular 3 to 8, such as ethyl acetate, propyl acetate, butyl acetate, butyl formiate, methyl acetate, methyl propionate, methyl butyrate, ethyl propionate and ethylbutyrate. When using these less polar solvents, re-crystallization is preferably performed as a crystallization from a suspension in order to reduce the amount of solvents.

Both the preferential crystallization and the re-crystallization may be carried out as a batch process or as a continuous process. One means for carrying out the process continuously is illustrated in figure 1 of WO 97/32644, to which full reference is made.

The mother liquor obtained in the first crystallization according to the present invention is depleted with regard to the desired enantiomer and thus contains an excess of the other or non-desired enantiomer. In a preferred embodiment of the invention, said mother liquor is subjected to a further crystallization in order to crystallize the acid-addition salt of the non-desired enantiomer, i.e. if the acid addition salt obtained in the first crystallization is enriched with regard to *S*-PPA, then crystalliziation from the mother liquor yields the acid-addition salt of PPA which is enriched with regard to the R-enantiomer of PPA, and vice versa.

Thus a particular embodiment of the invention relates to a process comprising the following steps:
i) fractional crystallization of PPA in the form of its acid-addition salt with the achiral carboxylic acid A, whereby the acid-addition salt is obtained in the form of a crystalline material that is enriched with one enantiomer of PPA, preferably R-PPA, and removing the obtained crystalline material from the mother liquor;
ii) supersaturating the mother liquor remaining in step i) and effecting crystallization of the acid addition salt of the other enantiomer of PPA, preferably S-PPA, with the achiral acid A from the mother liquor, whereby the acid-addition salt in the form of a crystalline material that is enriched with the other enantiomer (preferably S-PPA) is obtained, and removing the obtained crystalline material from the mother liquor.

In order to achieve the crystallization in step ii) of the other enantiomer, the mother liquor obtained in step i) has to be supersaturated prior to crystallization in step ii). Supersaturation of the mother liquor may be achieved by further reducing the temperature of the mother liquor, by concentrating the mother liquor, or preferably by further adding or dissolving the mixture of the enantiomers of PPA and the achiral carboxylic acid A (either separately or as the acid-addition salt) to or in the mother liquor remained in step i). The crystallization of the other enantiomer (preferably. S-PPA) is then carried out as outlined above (step ii)). Thereby, the mother liquor is obtained which contains an excess of the one enantiomer. This mother liquor of step ii) can be subjected to a further crystallization as outlined above, preferably after addition or dissolving of the required amount of the mixture of enantiomers to be separated and the corresponding amount of achiral carboxylic acid A to or in the mother liquor remained in step ii). These steps of consecutive crystallization of the one and the other, in particular the R-enantiomer and the S-enantiomer, can be repeated until the yield is nearly quantitative. The process of consecutive crystallization allows a very efficient separation of the enantiomers of PPA and yield higher than 90 %, in particular higher than 95 % of theory can be achieved by a sufficient number of consecutive steps.

Thus a particular preferred embodiment of the invention relates to a process comprising the following steps:
i) fractional crystallization of PPA in the form of its acid-addition salt with the achiral carboxylic acid A, whereby the acid-addition salt is obtained in the form of a crystalline material that is enriched with one enantiomer, preferably R-PPA, and removing the obtained crystalline material from the mother liquor;
ii) supersaturating the mother liquor remaining in step i) and effecting crystallization of the acid addition salt of the other enantiomer of PPA, preferably S-PPA with the achiral acid A from the mother liquor, whereby the acid-addition salt in the form of a crystalline material that is enriched with the other enantiomer (preferably S-PPA) is obtained, and removing the obtained crystalline material from the mother liquor;
iii) supersaturating the mother liquor remaining in step ii) and effecting crystallization of the acid addition salt of the one enantiomer of PPA (preferably R-PPA), whereby the acid-addition salt in the form of a crystalline material that is enriched with the one enantiomer is obtained, and removing the obtained crystalline material from the mother liquor; and
optionally repeating steps ii) and iii) once or several times.

The acid addition salt of PPA with the achiral acid A, in particular the acid addition salts of PPA with the achiral acid A' and especially the acid addition salt of PPA with 3,5-DNTA or with 3,5-DNBA are valuable intermediates in obtaining enantiomerically enriched PPA and thus form also part of the present invention. In these salts the molar ratio of PPA to achiral acid A (or A', respectively) is close to stoichiometry, but small deviations are possible. Usually the molar ratio of PPA to achiral acid A (or A', respectively) in these salts is from 0.9:1 to 1.1:1, in particular from 0.95:1 to 1.05:1.

The acid addition salts of PPA with the achiral acid A, in particular the acid addition salts of PPA with the achiral acid A' and especially the acid addition salt of PPA with 3,5-DNTA or with 3,5-DNBA may be racemic or close to the racemate, i.e. the enantiomeric excess with regard to R-PPA or S-PPA is < 20 % ee. The acid addition salts of PPA with the achiral acid A, in particular the acid addition salts of PPA with the achiral acid A' and especially the acid addition salt of PPA with 3,5-DNTA or with 3,5-DNBA may, however be enatiomerically enriched with regard to either the R-PPA or the S-PPA. In this case, the enantiomeric excess exceeds 20 % ee and is generally at least 65:35 (ee = 30 %), frequently at least 70 : 30 (ee = 40 %), in particular at least 75:25 (ee = 50 %) or at least 80 : 20 (ee = 60%). However, a higher enantiomeric excess can also be achieved.

The acid addition salts of PPA with achiral acid A may be present in pure form, i.e. the impurities different from PPA and achiral acid A make up at most 1 % by weight, in particular at most 0.5 % by weight or at most 0.1 % by weight of the total weight of the acid addition salt. However the acid addition salts of PPA with PCPA may be present in the form of solvates which are also part of the present invention.

The desired enantiomer of the acid-addition salt of PPA can be transformed into its free base as described in the prior art cited in the introductory part of the present application. The preparation of the free base is usually achieved by treatment with a diluted aqueous base, in particular by treatment with an aqueous solution of an alkalimetal carbonate, alkalimetal hydrogen carbonate or alkalimetal hydroxide such as sodium carbonate, potassium carbonate, sodium hydrogencarbonate, sodium hydroxide, calcium hydroxide or potassium hydroxide, optionally in the presence of an organic solvent to assist solubilization of the free base. Optionally, the carboxylic acid can be removed with a basic ion exchange resin. Likewise, it is possible to remove PPA with an acidic ion exchange resin from which it can be isolated by elution with an acid such as dilute hydrochloric acid or dilute sulphuric acid. It is also possible to recover the achiral acid A by adding an aqueous solution of an acid stronger than the achiral acid A (i.e. an acid having a pKs lower than the pKs of the achiral acid A), in particular an aqueous solution of an inorganic acid such as hydrochloric acid, thereby liberating the achiral acid and transform the PPA into its acid addition salt with the stronger acid. The achiral acid A can then be separated from the thus obtained aqueous mixture by filtration or extraction and PPA can be isolated as its acid addition salt with the stronger acid or by extraction under alkaline conditions.

The thus obtained free base of the S-enantiomer may then be reacted to Repaglinide by the methods described in prior art, e.g. by the methods described in EP 99017, EP 589874, EP 2019097 or WO 2009/004485.

The process of the invention allows a very efficient resolution of racemic PPA into its enantiomers S-PPA and R-PPA with high enantiomeric ratios of > 95:5, in particular > 98:2, especially > 99:1 or even > 99.9:0.1. While S-PPA (or PPA enriched with regard to S-PPA) is of high interest, the process also yields large amounts of R-PPA (or PPA enriched with regard to R-PPA), which is commercially less valuable.

Therefore, it is of interest to subject R-PPA or PPA enriched with regard to R-PPA to a racemisation and then to subject the racemate to a process according to the present invention in order to obtain further S-PPA. It was now found that racemisation can be achieved by standard methods for racemisation of benzylic amines. Therefore a particular embodiment of the invention relates to a process which additionally comprises racemisation of the non-racemic 3-methyl-1-(2-piperidinophenyl)-1-butylamine, which is enriched with the R enantiomer of 3-methyl-1-(2-piperidinophenyl)-1-butylamine.

Usually the racemisation of non-racemic PPA is performed in the presence of a racemisation catalyst, which is generally a basic compound. Suitable catalysts for racemisation of non-racemic PPA include but are not limited to alkalimetals, in particular lithium or sodium, which may be supported on an inert carrier such as aluminium oxide, silicium dioxide or alkalimetal carbonate, alkali metal compounds of polycyclic aromatic compounds such as lithium or sodium compounds of naphthalene, anthracene, phenantrene, di-tert-butyl-biphenyl, in particular lithium or sodium naphthalide, alkalimetal alkoxides such as sodium or potassium alkoxides, alkalimetal hydroxides, e.g. NaOH or KOH in dimethylsulfoxide, alkalimetal hydrides such as sodium hydride, alkalimetal amides such as NaNH₂, alkalimetal alkylamides and alkyl-alkalimetal compounds, in particular alkyl-lithium compounds such as butyl-lithium. Racemization may also be achieved by treatment of non-racemic PPA with hydrogen in the presence of a hydrogenation catalyst such as Raney nickel.

Racemisation of non-racemic PPA can be performed by standard techniques, which have been described in the art, e.g. US 4,158,016, US 2,152,976, US 2,797,243, US 3,168,566, US 3,954,870, US 3,970,700, US 4,246,424, US 4,252,744, US 4,777,291, US 5183939 and EP 900780.

Those skilled in the art will appreciate that the invention described herein is susceptible to variations and modifications other than those specifically described. The invention includes all such variation and modifications. The invention also includes all of the steps, features, formulations and compounds referred to or indicated in the specification, individually or collectively and any and all combinations or any two or more of the steps or features.

Each document, reference, patent application or patent cited in this text is expressly incorporated herein in their entirety by reference, which means that it should be read and considered by the reader as part of this text. That the document, reference, patent application or patent cited in this text is not repeated in this text is merely for reasons of conciseness. None of the cited material or the information contained in that material should, however be understood to be common general knowledge.

Manufacturer's instructions, descriptions, product specifications, and product sheets for any products mentioned herein or in any document incorporated by reference herein, are hereby incorporated herein by reference, and may be employed in the practice of the invention.

The present invention is not to be limited in scope by any of the specific embodiments described herein. These embodiments are intended for the purpose of exemplification only. Functionally equivalent products, formulations and methods are clearly within the scope of the invention as described herein.

The invention described herein may include one or more range of values (e.g. size, concentration etc). A range of values will be understood to include all values within the range, including the values defining the range, and values adjacent to the range which lead to the same or substantially the same outcome as the values immediately adjacent to that value which defines the boundary to the range.

Throughout this specification, unless the context requires otherwise, the word "comprise" or variations such as "comprises" or "comprising", will be understood to imply the inclusion of a stated integer or group of integers but not the exclusion of any other integer or group of integers.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs.

In the following examples all % values are to be understood as % by weight.

### Abbreviations:

| | |
|---|---|
| NMP: | N-methyl pyrrolidone |
| MEK: | Methyl ethyl ketone |
| MeOH: | Methanol |
| EtOH: | Ethanol |
| TBME: | tert.-butylmethyl ether |
| 3,5-DNTA: | 4-methyl-3,5-dinitrobenzoic acid |
| 3,5-DNBA: | 3,5-dinitrobenzoic acid |
| *R*-PPA: | (1R)-3-methyl-1-(2-piperidin-1-ylphenyl)butylamine |
| *S*-PPA: | (1S)-3-methyl-1-(2-piperidin-1-ylphenyl)butylamine |
| *rac*-PPA: | racemic 3-methyl-1-(2-piperidin-1-ylphenyl)butylamine |
| N-acetylated PPA: | N-[3-methyl-1-(2-piperidin-1-ylphenyl)-1-butyl] acetamide |
| Surfactant 1: | Polyoxyethylene (10) nonylphenyl ether (HLB >10): Synperonic NP10 |
| S/R | enantiomeric ratio of *S*-PPA / *R*-PPA |

### Analytics:

The enantiomeric ratio S/R was measured via chiral HPLC of the N-acetylated PPA:
column: Chiralpak AD 250*4.6; eluent: hexane/isopropyl acetate 95:5; detector: UV 220 nm; 20°C.

Optical rotation were measured at 20°C by means of a polarimeter (Knauer) at λₘₐₓ = 589 nm.

### Preparation Example 1: Preparation of rac-PPA*3,5-DNTA

11.3 g 3,5-DNTA and 12.3 g *rac*-PPA were stirred for 2 h in 50 ml of methyl ethyl ketone. The yellow crystals of *rac*-PPA*3,5-DNTA were filtered off and dried.

The thus obtained acid addition salt had the overall composition (PPA)(3,5-DNTA)ₘ with m being close to 1.

### Preparation Example 2: Preparation of rac-PPA*3,5-DNBA

By analogy to preparation example 1, the acid addition salt *rac*-PPA*3,5-DNBA was prepared. The thus obtained acid addition salt had the overall composition (PPA)(3,5-DNBA)ₘ with m being close to 1.

### Example 1: Resolution via entrainment of rac-PPA*3,5-DNTA

20.00 g *rac*-PPA*3,5-DNTA were dissolved at 70 °C in a mixture of 60 g NMP, 10 g isobutyl alcohol, 10 g of surfactant 1 and 20 g of water. The homogenous solution was cooled to 28 °C and seeded with 100 mg of *R*-PPA*3,5-DNTA (enantiomeric ratio R/S > 99:1). The mixture was shaken for 7 min and filtered with suction through a sintered glass funnel. The obtained yellow crystals (2.80 g) showed the following optical rotation: - 3.35 (c = 20 mg/ml, MeOH) (S/R-ratio 30:70).

The above mother liquor was heated at 70 °C until a homogenous solution was obtained. The solution was cooled to 28 °C and seeded with 100 mg *S*-PPA*3,5-DNTA. The mixture was shaken for 10 min and filtered under suction through a sintered glass funnel. The yellow crystals (1.50 g) showed the following optical rotation: + 6,85 (c = 20 mg/ml, MeOH). (S/R-ratio 91:9)

The above mother liquor was heated at 70 °C until a homogenous solution was obtained. The solution was cooled to 28 °C and seeded with 100 mg *R*-PPA*3,5-DNTA. The mixture was shaken for 30 min and filtered under suction through a sintered glass funnel. The yellow crystals (1.57 g) showed the following optical rotation: - 4.35 (c = 20 mg/ml, MeOH). (S/R-ratio 24:76)

To the above mother liquor were added 1.87 g *rac*-PPA*3,5-DNTA and the suspension was heated to 70 °C until a homogenous solution was obtained. The solution was cooled to 28 °C and seeded with 100 mg *S*-PPA*3,5-DNTA. The mixture was shaken for 20 min and filtered under suction through a sintered glass funnel. The yellow crystals (900 mg) showed the following optical rotation: + 7.85 (c = 20 mg/ml, MeOH). (S/R-ratio 97:3)

Starting from a total amount of 27.3 g of racemic PPA, the total yield from eight consecutive runs (as above, with replenished *rac*-PPA) were 6.45 g *R*-enriched PPA with an enatiomeric ratio S/R of about 20 : 80 and 5.40 g *S*-enriched PPA with an enatiomeric ratio S/R of about 93 : 7.

### Example 2: Resolution via entrainment with 3,5-DNBA

246 mg *rac*-PPA and 424 mg 3,5-dinitrobenzoic acid were dissolved at 70 °C in a mixture of 60 % ethanol, 10 % isobutyl alcohol, 10 % surfactant 1 and 10 % water (3,0 ml). The solution was cooled to 22 °C and seeded with 3.8 mg *S*-PPA*3,5 DNBA. The mixture was shaken for 16 h and the crystals were filtered off. The crystals were washed with EtOH (0,5 ml) and dried. The yield was 12,0 mg and and the enantiomeric ratio S/R was 96.0 : 4.0.

### Example 3: Resolution via entrainment with 3,5-DNTA

369 mg *rac*-PPA and 675 mg 4-methyl-3,5-dinitrobenzoic acid were dissolved at 80 °C in a mixture of 10 % NMP, 75 % octyl alcohol, 10 % surfactant 1 and 5 % water (3.3 ml). The solution was cooled to 22 °C and seeded with 3.0 mg *S*-PPA*3,5-DNTA. The mixture was shaken for 10 min and the crystals were filtered off. The crystals were washed two times with isobutyl alcohol (0.5 ml) and dried. The yield was 60.0 mg and the enantiomeric ratio S/R was 95.2 : 4.8.

### Example 4: Resolution via entrainment with 3,5-DNTA

3009 mg of the acid addition salt *rac*-PPA*3,5-DNTA were dissolved at 80 °C in a mixture of 10 % NMP, 75 % octyl alcohol, 10 % surfactant 1 and 5 % water (20.0 ml). The solution was cooled to 19 °C and seeded with10.0 mg *S*-PPA*3,5-DNTA. The mixture was shaken for 6 min and the crystals were filtered off. The crystals were washed two times with isopropyl alcohol (2.5 ml) and dried. The yield was 485.0 mg and the enantiomeric ratio S/R was 97.0 : 3.0.

### Example 5: Enhancement of the optical purity of S-PPA*3,5-DNTA

435 mg *S*-PPA*3,5-DNTA with an enantiomeric ratio of 97.0 : 3.0, obtained e.g. from Example 4, were suspended with stirring in isopropyl alcohol (4.0 ml) at 22 °C for 2 h. Then the crystals were filtered off, washed two times with isopropyl alcohol (0.5 ml) and dried. The yield was 378.0 mg and the enantiomeric ratio S/R was 98.0 : 2.0.

358 mg *S*-PPA*3,5-DNTA with a S/R ratio of 98.0 : 2.0 were heated at 70 °C in isopropyl alcohol (4.0 ml). The solution was cooled to 22 °C and stirred for 2 h. The yellow crystals were filtered off and washed two times with isopropyl alcohol (0.5 ml) and dried. The yield was 290 mg and the enantiomeric ratio was 99.7 : 0.3 (S / R), measured via chiral HPLC.

### Example 6: Enhancement of the optical purity of S-PPA*3,5-DNTA (2)

7 g of *S*-PPA*3,5-DNTA having a S/R ratio of 89 : 11 were suspended in MEK (21 g) and stirred for 15 h at 20 °C. The obtained crystalline material was separated off and dried in vacuo to yield 5.73 g of pure acid addition salt of PPA with 3,5-DNTA which showed an enantiomeric ratio S/R of 99 : 1.

The thus obtained acid addition salt had the overall composition (PPA)(3,5-DNTA)ₘ with m being close to 1.

### Example 7: Enhancement of the optical purity of S-PPA*3,5-DNTA (3)

154 g of *S*-PPA*3,5-DNTA having a S/R ratio of 98.6 : 1.4 were suspended in MEK (220 g) and stirred for 1 h at 50°C and then for 6 h at 18 °C. The obtained crystalline material was separated off and dried in vacuo to yield 98 g of pure acid addition salt of PPA with 3,5-DNTA which showed an enantiomeric ratio S/R of 99.97 : 0.03.

The thus obtained acid addition salt had the overall composition (PPA)(3,5-DNTA)ₘ with m being close to 1.

### Comparative Example: Resolution with malic acid

2000 mg *rac*-PPA and 1088 mg L-malic acid were stirred together for 15 h at 12 °C in TBME (5 ml). The precipitated salt (1575 mg) was isolated and washed three times with TBME (2 ml) and showed an enantiomeric ratio of 42.9 : 57.1 (S / R). 1500 mg of this salt were stirred for 1 h at 22 °C in aceton (7.5 ml). The precipitated salt (1366 mg) was isolated and washed three times with TBME (2 ml) and showed an enantiomeric ratio S/R of 38.6 : 61.4.

### Example 8: Racemisation of R-PPA

To 1.232 g of enantiomeric enriched *R*-PPA (enantiomeric ratio S/R 3.6 : 96.4) was added a solution of sodium naphthalene (20 mol %) in THF (250 µl) under argon. The solution was held at 80 °C for 60 h, cooled to 23 °C and diluted with TBME (5 ml) and hydrochloric acid (1 N, 12 ml). The organic layer was discharged, the water layer was basified with solid sodium hydroxide and extracted with TBME (3 x 5 ml). After drying (MgSO₄) and evaporation of the organic phase, pure PPA (994 mg) with an enantiomeric ratio S/R of 41 : 59 was obtained.

## Claims

1. A process for enantiomeric enrichment of 3-methyl-1-(2-piperidinophenyl)-1-butylamine, the process comprising the fractional crystallization of 3-methyl-1-(2-piperidinophenyl)-1-butylamine in the form of its acid-addition salt with an achiral carboxylic acid A from a solution or emulsion containing a mixture of the enantiomers of 3-methyl-1-(2-piperidinophenyl)-1-butylamine and the achiral carboxylic acid A, whereby the acid-addition salt of 3-methyl-1-(2-piperidino-phenyl)-1-butylamine with the achiral carboxylic acid A is obtained, which is enantiomerically enriched with regard to one enantiomer of 3-methyl-1-(2-piperidinophenyl)-1-butylamine,
where the achiral carboxylic acid A is a compound or a mixture of compounds of the formula A 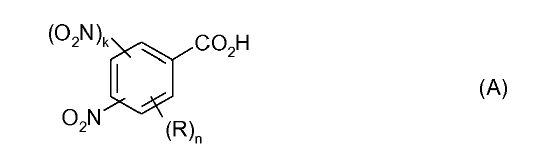 wherein n is 0 or 1, k is 0 or 1 and wherein R is C₁-C₄-alkyl, C₁-C₄-alkoxy and halogen.

2. The process according to claim 1, wherein the carboxylic acid A is selected from 3,5-dinitrobenzoic acid, which optionally carries a radical R.

3. The process according to claim 2, wherein the carboxylic acid A is selected form 3,5-dinitrobenzoic acid and 4-methyl-3,5-dinitrobenzoic acid.

4. The process according to any of the preceding claims, wherein 3-methyl-1-(2-piperidinophenyl)-1-butylamine is present in the solution before crystallization as mixture of its enantionmers having an enantiomeric excess of less than 20 % ee.

5. The process according to any of the preceding claims, wherein the crystallization is effected from a supersaturated solution of the acid-addition salt in a solvent, selected from water, water-miscible organic solvents and mixtures thereof.

6. The process according to any of claims 1 to 4, wherein the crystallization is effected from a supersaturated emulsion.

7. The process according to any of the preceding claims, wherein the crystallization is effected by addition of seed crystals of the acid addition salt of one enantiomer of 3-methyl-1-(2-piperidinophenyl)-1-butylamine with the achiral carboxylic acid A to a solution or emulsion containing a mixture of the enantiomers of 3-methyl-1-(2-piperidinophenyl)-1-butylamine and the achiral carboxylic acid A.

8. The process according to any of the preceding claims, where the mother liquor obtained from crystallization of the acid-addition salt of 3-methyl-1-(2-piperidinophenyl)-1-butylamine with the achiral carboxylic acid A is subjected to a second crystallization to obtain the acid-addition salt of 3-methyl-1-(2-piperidino-phenyl)-1-butylamine with the achiral carboxylic acid A, which is enantiomerically enriched with regard to the other enantiomer.

9. The process according to any of the preceding claims, comprising
i) fractional crystallization of 3-methyl-1-(2-piperidinophenyl)-1-butylamine in the form of its acid-addition salt with the achiral carboxylic acid A, whereby the acid-addition salt is obtained in the form of a crystalline material that is enriched with one enantiomer of 3-methyl-1-(2-piperidinophenyl)-1-butylamine, and removing the obtained crystalline material from the mother liquor;
ii) supersaturating the mother liquor remaining in step i) and effecting crystallization of the acid addition salt of the other enantiomer of 3-methyl-1-(2-piperidinophenyl)-1-butylamine with the achiral acid A from the mother liquor, whereby the acid-addition salt in the form of a crystalline material that is enriched with the other enantiomer is obtained, and removing the obtained crystalline material from the mother liquor.

10. The process according to claim 9, additionally comprising
iii) supersaturating the mother liquor remaining in step ii) and effecting crystallization of the acid addition salt of the one enantiomer of 3-methyl-1-(2-piperidinophenyl)-1-butylamine, whereby the acid-addition salt in the form of a crystalline material that is enriched with the one enantiomer is obtained, and removing the obtained crystalline material from the mother liquor;
and
optionally repeating steps ii) and iii) once or several times.

11. The process according to any of the preceding claims, which comprises racemisation of the non-racemic 3-methyl-1-(2-piperidinophenyl)-1-butylamine, which is enriched with the R enantiomer of 3-methyl-1-(2-piperidinophenyl)-1-butylamine.

12. The process of claim 11, where the racemisation is achieved by reacting the non-racemic 3-methyl-1-(2-piperidinophenyl)-1-butylamine in the presence of a racemisation catalyst which is selected from alkalimetals, alkali metal compounds of polycyclic aromatic compounds, alkalimetal alkoxides, alkalimetal hydroxides, alkalimetal hydrides, alkalimetal amides, alkalimetal alkylamides and alkyl-alkalimetal compounds.

13. The process according to any of the preceding claims, further comprising a recrystallization of the obtained enantiomerically enriched acid-addition salt.

14. The process according to any of the preceding claims, further comprising the transformation of the obtained enantiomerically enriched acid-addition salt into the free base of 3-methyl-1-(2-piperidinophenyl)-1-butylamine.

15. The acid-addition salt of 3-methyl-1-(2-piperidinophenyl)-1-butylamine with the achiral carboxylic acid A as defined in claim 1.
